# EUROPEAN PATENT APPLICATION

(11) **EP 4 806 977 A1**
(43) Date of publication of application: **16.09.2026**
(21) Application number: 24917247.9
(22) Date of filing: 01.08.2024
(51) Int. Cl.: C10G 2/00, C10J 3/72, C01B 3/38, C01B 3/48, C01B 32/40, C07C 1/20, C07C 29/151, C07C 31/04

(54) **METHOD AND APPARATUS FOR PRODUCING HYDROCARBONS**

(30) Priority: 11.01.2024 KR 20240004571
(71) Applicant: SK Innovation Co., Ltd., Seoul 03188 (KR)
(72) Inventor: JEON, Heejung, Daejeon 34124 (KR); SON, Sungreal, Daejeon 34124 (KR); YU, Seunghan, Daejeon 34124 (KR); JEONG, Yongseong, Daejeon 34124 (KR)
(74) Representative: Lorenz Seidler Gossel Part. mbB
(86) International application number: PCT/KR2024/095960
(87) International publication number: WO 2025/150758

(57) **Abstract**

Disclosed is a method for producing hydrocarbons, the method comprising the steps of: S1) thermally decomposing a mixed waste to produce a first mixed gas; S2) steam-reforming the first mixed gas, removed of impurities, in a first fluidized bed reactor to produce a second mixed gas; S3) separating the second mixed gas into a first stream containing carbon dioxide and a second stream containing hydrogen and carbon monoxide; S4) introducing the first stream, separated in step S3), into a second fluidized bed reactor and converting the first stream into carbon monoxide through a reverse Boudouard reaction; S5) mixing the second stream and the carbon monoxide, converted in step S4), to produce a third mixed gas, and producing a synthesis gas (Syngas) through a water-gas conversion reaction using the third mixed gas; and S6) producing hydrocarbons from the synthetic gas through a catalytic reaction.

## Description

### [Technical Field]

The present disclosure relates to a method and an apparatus for producing a hydrocarbon from an organic waste, and more particularly, to a method and an apparatus for producing a hydrocarbon which may improve a hydrocarbon conversion yield, using a catalyst having high activity and durability in a reforming reaction of a mixed gas including impurities.

### [Background Art]

Organic wastes may seriously damage the environment due to decomposition when landfilled and should be discarded through a prescribed treatment process after collecting them according to their properties when discarded. However, since simple disposal of the organic wastes requires securing treatment facilities and consuming a large amount of manpower and is more wasteful than productive, methods and technologies for recycling organic wastes have been developed in recent years. Representatively, a gasification process technology in which synthetic gas is produced using organic wastes and converted into a high value-added product for energization may be mentioned.

The gasification process generally refers to a series of processes of reacting carbonaceous raw materials such as coal, organic wastes, and biomass under the supply of water vapor, oxygen, carbon dioxide, or a mixture thereof to convert the raw materials into synthetic gas including hydrogen and carbon monoxide, in which the "synthetic gas" refers to a mixed gas which is usually produced by a gasification reaction and includes hydrogen and carbon monoxide and may further include carbon dioxide and/or methane.

The gasification process technology has expanded to a technology of producing fuels and raw materials of various compounds, and for example, the synthetic gas may be used as the raw material of a Fischer-Tropsch synthesis reaction to manufacture high value-added products such as light oil, heavy oil, diesel oil, wax, jet oil, and lubricant base oil. Besides, it is known that the technology may be applied to hydrogen power generation, ammonia manufacture, an oil refining process, and the like, using hydrogen in the synthetic gas which is the main product of the gasification process, and high value-added chemicals such as acetic acid, olefin, dimethyl ether, aldehyde, fuel, and an additive may be obtained, using methanol manufactured from the synthetic gas. However, since the synthetic gas produced from organic wastes has a very poor production yield, it is difficult to effectively produce a high value-added compound material from the synthetic gas.

Recently, as a process for manufacturing synthetic gas, a gasification process using a catalyst has been carried out, but the catalyst is deactivated by the formation of coke and the like in the gasification process, and process trouble occurs due to the deactivated catalyst during a continuous operation. In addition, for securing economic feasibility, relatively expensive catalysts need to be recovered, but in order to recover catalyst discharged in the state in which coke and the like are agglomerated, a plurality of subsequent processes (such as air burning) should be carried out, and thus, process efficiency is significantly deteriorated. In addition, since a mixed gas obtained by pyrolyzing an organic waste includes water-soluble impurities such as H₂S, HCl, HOCl, and NH₃ and water-insoluble impurities such as tar, in addition to hydrogen, carbon monoxide, and carbon dioxide, inactivation of a catalyst used in a subsequent process of producing synthetic gas is induced to lower the efficiency of the subsequent process, and when the mixed gas is purified due to the problem, the efficiency of the entire process may be lowered.

In addition, since the conventionally performed gasification process of organic wastes has a significantly low production yield of synthetic gas, which may be converted into a high value-added product, of 30% or less, its productivity is poor, and there are limitations to utilizing or commercializing the process. Further, in terms of environmental protection, it is preferred to suppress CO₂ emission, but since the gasification reaction product of organic wastes contains CO₂ in addition to H₂ and CO, more carbon dioxide is emitted as compared with the case of landfill or pyrolysis treatment, and thus, the gasification process has a serious problem of causing rather increased environmental pollution.

Thus, development of a method and an apparatus for producing a hydrocarbon which may selectively and effectively remove the various impurities included in the pyrolysis mixed gas of the organic waste to improve the manufacturing yield of the synthetic gas, thereby performing efficient conversion into a high value-added hydrocarbon, while minimizing occurrence of carbon dioxide is still requested.

### [Disclosure]

### [Technical Problem]

An object of the present disclosure is to provide a method for producing a hydrocarbon by reforming a mixed gas including impurities such as Cl, S, and N recovered in a gasification process of a waste.

Another object of the present disclosure is to provide a method and an apparatus for producing a hydrocarbon, including a catalyst having improved reaction activity and durability even in a mixed gas including impurities.

Another object of the present disclosure is to provide a method and an apparatus for producing a hydrocarbon, which are environmentally friendly even to a mixed gas including impurities and may stably reform a mixed gas.

Still another object of the present disclosure is to provide a method and an apparatus for producing a hydrocarbon, which may be used in recycling of eco-friendly chemical raw materials for preventing climate change and the like, by suppressing pollution and greenhouse gas emissions, since a high value-added synthetic gas is produced from an organic waste.

### [Technical Solution]

In one general aspect, a method for producing a hydrocarbon includes: S1) pyrolyzing a mixed waste to produce a first mixed gas; S2) steam-reforming the first mixed gas from which impurities have been removed in a first fluidized bed reactor to produce a second mixed gas; S3) separating the second mixed gas into a first stream including carbon dioxide and a second stream including hydrogen and carbon monoxide; S4) introducing the first stream separated in S3) into a second fluidized bed reactor and converting the first stream into carbon monoxide through a reverse Boudouard reaction; S5) mixing the second stream and the carbon monoxide converted in S4) to produce a third mixed gas and treating the third mixed gas through a water gas conversion reaction to produce a synthetic gas (syngas); and S6) producing a hydrocarbon from the synthetic gas through a catalytic reaction.

In an example, S2) and S4) may be performed under a catalyst containing a first metal including a Group VIIIA element, a Group VIA element, or a combination thereof; and a second metal including an alkaline earth metal oxide or an alkaline earth metal oxide structure.

In an example, the first mixed gas may further include one or more selected from the group consisting of landfill gas, shale gas, refinery exhaust gas, and biogas.

In an example, the second fluidized bed reactor in S4) may be supplied with a coking catalyst of the first fluidized bed reactor of S2) as a carbon source.

In an example, the first stream may include 50 vol% or more of carbon dioxide.

In an example, the pyrolysis gas may have a C/O element ratio of 0.1 or more.

In an example, S2) may be performed at 700°C to 1000°C.

In an example, S4) may be performed at a temperature of 600°C to 1000°C and a pressure of 50 KPa to 300 KPa.

In an example, the synthetic gas in S5) may include hydrogen and carbon monoxide, and a ratio between the hydrogen and the carbon monoxide may satisfy 1.8:1 to 2.2:1.

In an example, the organic wastes in S1) may be one or more selected from the group consisting of waste plastic, solid waste, biomass, waste oil, waste tire, and standard plastic garbage bags.

In another general aspect, an apparatus for producing a hydrocarbon is provided.

In another general aspect, an apparatus for producing a hydrocarbon includes: a pyrolysis reactor which heat treats an organic waste to produce a first mixed gas; a first fluidized bed reactor which steam-reforms the first mixed gas to produce a second mixed gas; a carbon dioxide separation unit which separates the second mixed gas into a first stream including carbon dioxide and a second stream including hydrogen and carbon monoxide; a second fluidized bed reactor which converts the first stream into carbon monoxide through a reverse Boudouard reaction; a gas mixing unit which mixes the second stream and carbon monoxide converted in the second fluidized bed reactor to produce a third mixed gas; a synthetic gas production unit which converts the third mixed gas into a synthetic gas through a water gas conversion reaction; and a hydrocarbon conversion unit which converts the synthetic gas into a hydrocarbon through a catalytic reaction.

In an example, the first fluidized bed reactor and the second fluidized bed reactor may include a catalyst containing a first metal including a Group VIIIA element, a Group VIA element, or a combination thereof in the periodic table; and a second metal including an alkaline earth metal oxide or an alkaline earth metal oxide structure.

In an example, the apparatus for producing a hydrocarbon may further include a cyclone connected between the first fluidized bed reactor and the carbon dioxide separation unit; a catalyst supply line which connects the cyclone and the second fluidized bed reactor; and a catalyst recirculation line which connects the second fluidized bed reactor and the first fluidized bed reactor, wherein the cyclone separates the second mixed gas and the catalyst discharged from the fluidized bed reforming reactor, supplies the second mixed gas to the carbon dioxide separation unit, and supplies the catalyst to the second fluidized bed reactor.

### [Advantageous Effects]

According to an exemplary embodiment of the present disclosure, a catalyst having excellent reaction activity and durability is introduced, thereby producing a hydrocarbon with high efficiency without rapid deactivation of the catalyst even for a mixed gas including impurities,

According to an exemplary embodiment of the present disclosure, a mixed gas including impurities such as Cl, N, and S may be environmentally friendly and stably reformed.

According to an exemplary embodiment of the present disclosure, since conversion into a hydrocarbon may be performed using a process and an apparatus appropriate for a C/O element ratio of a feed, the manufacturing efficiency of a hydrocarbon may be significantly improved.

### [Description of Drawings]

FIG. 1 is a schematic diagram showing an apparatus for producing a hydrocarbon according to an example of the present disclosure.
FIG. 2 is a schematic diagram showing an apparatus for producing a hydrocarbon including a purification process according to an example.
FIG. 3 is a schematic diagram showing an apparatus for producing a hydrocarbon including a catalyst circulation process according to an example.

### [Best Mode]

Hereinafter, the present disclosure will be described in detail with reference to the accompanying drawings. However, it is only illustrative and the present disclosure is not limited to the specific embodiments which are illustratively described in the present disclosure.

The singular form of the term used herein may be intended to also include a plural form, unless otherwise indicated.

The numerical range used in the present specification includes all values within the range including the lower limit and the upper limit, all double limited values, and all possible combinations of the upper limits and the lower limits in the numerical range defined in different forms. Unless otherwise defined in the present specification, values which may be outside a numerical range due to experimental error or rounding off of a value are also included in the defined numerical range.

The term "comprise" mentioned in the present specification is an open-ended description having a meaning equivalent to the term such as "is/are provided", "contain", "have", or "is/are characterized", and does not exclude elements, materials, or processes which are not further listed.

The unit of % used in the present specification without particular mention refers to % by weight, unless otherwise defined.

The terms "Group VIIIA element" and "Group VIA element" used in the present specification and attached claims refer to elements corresponding to each group in the periodic table according to the old IUPAC (International Union of Pure and Applied Chemistry).

The present disclosure includes a method for producing a hydrocarbon including: S1) pyrolyzing a mixed waste to produce a first mixed gas; S2) steam-reforming the first mixed gas in a first fluidized bed reactor to produce a second mixed gas; S3) separating the second mixed gas into a first stream including carbon dioxide and a second stream including hydrogen and carbon monoxide; S4) introducing the first stream separated in S3) into a second fluidized bed reactor and converting the first stream into carbon monoxide through a reverse Boudouard reaction; S5) mixing the second stream and the carbon monoxide converted in S4) to produce a third mixed gas and treating the third mixed gas through a water gas conversion reaction to produce a synthetic gas (syngas); and S6) producing a hydrocarbon from the synthetic gas through a catalytic reaction.

S2) and S4) of the present disclosure are performed in a fluidized bed reactor, and more specifically, the fluidized bed reactor may perform a reaction using a catalyst including a first metal including a Group VIIIA element, a Group VIA element, or a combination thereof in the periodic table and a second metal oxide including an alkaline earth metal oxide or a structure thereof to further remove impurities, and also simultaneously increase efficiency of a synthetic gas produced through a catalytic reaction.

Therefore, since the method for producing a hydrocarbon according to the present disclosure may efficiently remove impurities to prevent catalyst contamination or deactivation in a catalytic process, an organic waste may be converted into a synthetic gas with high efficiency as compared with a conventional gasification process, and the yield of a high value-added hydrocarbon converted from the synthetic gas may be maximized. In addition, carbon dioxide emissions may be minimized in a hydrocarbon production process to prevent environmental pollution.

In the present disclosure, S1) is a step of heat treating an organic waste to produce a first mixed gas, in which the gasification reaction of the organic waste occurs.

In an example, the organic waste in S1) may be one or more selected from the group consisting of waste plastic, solid waste, biomass, waste oil, waste tire, and standard plastic garbage bags.

Specifically, S1) is a gasification process of an organic waste, and may involve any one or more gasification reactions selected from the following Reaction Formulae 1 to 4:

[Reaction Formula 1] CₓH_{y} + H₂O → H₂ + CO (water gasification reaction)

[Reaction Formula 2] CₓH_{y} + CO₂ → CO (carbon dioxide gasification reaction)

[Reaction Formula 3] CO + 3H₂ → CH₄ + H₂O (methanation reaction)

[Reaction Formula 4] CₓH_{y} + O₂→ CO₂ (oxidation reaction).

In an example, the first mixed gas may include methane, hydrogen, carbon monoxide, and carbon dioxide, and may also include various impurities such as nitrogen oxides, sulfur oxides, and hydrogen chloride, for example, water soluble impurities such as H₂S, HCl, HOCl, and NH₃.

In an example, in order to increase the content of a dry gas in the composition of the first mixed gas, S1) may be performed under a catalyst condition or a non-catalyst condition. Herein, the dry gas is a common term for a hydrocarbon gas having 4 or fewer carbons. Since the efficiency of a methane reforming reaction may be increased by increasing the content of the dry gas in the composition of the first mixed gas, and it may be advantageous in terms of a synthetic gas production yield, an acid site catalyst or molybdenum-based forming catalyst may be used as a bed material of a pyrolysis reactor. When the catalyst is not used in the pyrolysis step, a methane content may be increased by operating the gasification pyrolysis reactor under the conditions of low temperature and high pressure. Since the yield improvement effect from methane reforming may be expected identically in a C2 to C4 hydrocarbon gas content as well as the methane content, the C2 to C4 gas content may also be increased by using the acid site catalyst or through a low temperature and high pressure gasification operation.

In an example, the catalyst in the pyrolysis reaction may be an acid site catalyst or molybdenum-based forming catalyst. In an example, the acid site catalyst may be alumina having a solid acid site or other inorganic structures. The alumina may be alumina alone, silica-alumina, alumina dispersed in a carbon structure, or the like. The structure may be zeolite, SAPO, AlPO, MOF, a structural variant thereof, or the like. The molybdenum-based catalyst refers to a catalyst in which molybdenum is supported on a support, and if necessary, may further include nickel, cobalt, and the like, or use tungsten instead of molybdenum. Any support may be used as long as it has durability against molybdenum or tungsten, and for example, may include materials including one or more selected from silica, alumina, silica-alumina, carbon, and zirconia.

As another example of increasing the methane content in the first mixed gas, the first mixed gas may further include one or more selected from the group consisting of landfill gas, shale gas, refinery exhaust gas, and biogas. Since the landfill gas, shale gas, refinery exhaust gas, and biogas include 40 vol% or more, specifically 50 vol% or more of methane and carbon dioxide, the first mixed gas further includes the gas described above, and thus, the production yield of synthetic gas may be further improved through a methane reforming reaction and a reverse Boudouard reaction which are subsequent processes.

In an example, the C/O element ratio of the first mixed gas may be 0.01 or more, 0.05 or more, or 0.1 or more and 0.9 or less, 0.8 or less, or 0.7 or less as the upper limit, and specifically may be 0.01 to 0.9, 0.05 to 0.8, and more specifically 0.1 to 0.7.

In the case in which the C/O element ratio of the first mixed gas satisfies the range described above, an oxygen content is higher than a carbon content in the first mixed gas when a methane reforming reaction described later is performed, and thus, an amount of coke produced by a side reaction is significantly decreased to prevent deactivation of the catalyst, thereby performing the methane reforming reaction with high efficiency.

After S1), an impurity gas including impurities such as Cl, N, and S in the mixed gas may be selectively removed using a scrubber or adsorbent. As an example, the impurities may be selectively removed by one or a combination of two or more selected from the group consisting of water washing, basic solution washing, scrubber, dust collection filter for high pressure, ceramic filter passing, and the like, but the present disclosure is not limited thereto. For example, among the means, when the first mixed gas passes through a ceramic filter, a dust collection filter, or the like, water-insoluble impurities such as tar, dusts, and the like may be removed.

S2) of the present disclosure is a step of steam reforming methane contained in the first mixed gas in a first fluidized bed reactor to produce a second mixed gas. In S2), a reforming reaction according to the following Reaction Formula 5 may be involved:

[Reaction Formula 5] CH₄ + H₂O → CO + 3H₂ (steam reforming reaction)

The reforming reaction of S2) may be performed at a temperature of 600°C to 1400°C, preferably 600°C to 700°C and a pressure of 30 KPa to 2000 KPa.

S2) may include a catalyst for increasing a reaction conversion rate. By adopting a catalyst including a first metal including a Group VIIIA element, a Group VIA element, or a combination thereof in the periodic table and a second metal oxide including an alkaline earth metal oxide or a structure thereof as the catalyst of S2), a fluidized bed catalyst reaction may proceed to further remove impurities and simultaneously increase the efficiency of a steam reforming reaction.

The first metal may include a Group VIIIA element, a Group VIA element, or a combination thereof, and for example, may include one or more selected from the group consisting of nickel (Ni), iron (Fe), molybdenum (Mo), and tungsten (W).

The second metal oxide may include an alkaline earth metal oxide or an alkaline earth metal oxide structure. The second metal oxide may include one or more alkaline earth metal oxide selected from the group consisting of beryllium (Be), magnesium (Mg), calcium (Ca), strontium (Sr), and barium (Ba), and for example, may include magnesium oxide (MgO). In addition, the alkaline earth metal oxide structure is a structure in which an alkaline earth metal is bonded to a metal oxide, and may include, for example, one or more selected from the group consisting of magnesium aluminate (MgAl₂O₄) having a spinel structure, hydrotalcite (Mg₆Al₂CO₃(OH)₁₆·4H₂O), and the like. In the present disclosure, since sulfur, nitrogen, and chlorine-based compounds may be selectively further removed by including the second metal oxide, the production efficiency of the synthetic gas in the steam reforming process of S2) and the reverse Boudouard process in S4) may be increased.

In addition, though the catalyst of the present disclosure including the first metal and second metal oxide performs catalyst coking and catalyst regeneration repeatedly, it has a small catalyst loss due to its excellent durability and may maintain high catalytic activity for a long time. As the catalyst exchange cycle is extended, process efficiency and economic feasibility may be improved.

Specifically, since the steam reforming reaction is performed in a fluidized bed reactor using the catalyst including two types of metals, reforming reactivity is improved, an effect of removing impurities such as chlorine is shown, side reactions including dehydrogenation and hydrogenation occur less, and thus, the yield of the produced synthetic gas may be maximized.

In an example, in the method for producing a catalyst, a ceramic support and the first metal may be mixed with a solvent to prepare a mixed solution, an organic acid may be added to the mixed solution to prepare a precursor gel, and then the precursor gel may be mixed with a solid mixture of a binder and the second metal oxide. An inorganic binder is further added to the mixture to prepare a composite catalyst sol, which may be then sprayed and dried to obtain a catalyst having an average particle diameter of 50 µm to 250 µm. The catalyst prepared by the method described above has excellent catalytic activity and also has improved durability since the first metal and the second metal are uniformly dispersed inside the catalyst and strongly bonded, thereby providing an excellent catalyst lifespan.

S3) is a step of separating the second mixed gas into a first stream including carbon dioxide and a second stream including hydrogen and carbon monoxide in a carbon dioxide separation unit.

In an example, a method for separating the second mixed gas into a first stream and a second stream is not limited as long as it is known in the art, but in the present disclosure, the separation may be performed using a carbon dioxide separation unit, and the carbon dioxide separation unit may be an amine scrubber. Usually, the amine scrubber binds and removes carbon dioxide through an amine-based material, and since it may separate components such as carbon dioxide and hydrogen sulfide from gas stream and recover gas containing hydrogen, carbon monoxide, or inert gas, when the amine scrubber is used, the first mixed gas may be separated into the first stream and the second stream.

As another example, the carbon dioxide separation unit may be a carbon capture and storage (CCS) unit. When the CCS unit is used for separation of carbon dioxide, it may adsorb and separate carbon dioxide using an adsorbent including any one or two or more selected from calcium oxide, calcium hydroxide, dolomite, limestone, or trona, and thus, the second mixed gas may be separated into the first and second streams using the carbon capture and storage (CCS) unit.

The first stream may include carbon dioxide at 40 vol% or more, 50 vol% or more, or 60 vol% or more as the lower limit and 99 vol% or less, 90 vol% or less, 80 vol% or less, or 70 vol% or less as the upper limit. Specifically, the first stream may include 40 to 99 vol%, more specifically 50 to 80 vol% of carbon dioxide. In the carbon dioxide separation unit, when carbon dioxide is captured and then separated, in order to separate carbon dioxide to a high purity, a regeneration tower in which carbon dioxide separation from an adsorbing material occurs should be designed with a high stage, and thus, more energy is consumed. Therefore, since the first stream includes carbon dioxide in the range described above, a carbon dioxide separation process may be performed under slightly milder conditions.

S4) is a step of converting the first stream separated in S3) into carbon monoxide through a reverse Boudouard reaction in a second fluidized bed reactor. Since carbon dioxide contained in the first stream is further converted into carbon monoxide through the reverse Boudouard reaction, environmental pollution may be prevented in terms of decreasing emitted carbon dioxide, while maximizing the yield of synthetic gas. The reverse Boudouard reaction may involve the following Reaction Formula 6:

[Reaction Formula 6] C+CO₂ → 2CO

The reaction may be performed at a temperature of 600°C to 1000°C and a pressure of 50 KPa to 300 KPa.

In addition, since a catalyst which is used in the steam reforming reaction and has coke deposited therein is used in the reverse Boudouard reaction, the process may be economically operated without supplying a separate carbon source from the outside. Since the catalyst regenerated by removing coke deposited in the catalyst in the reverse Boudouard reaction is added again to the steam reforming reaction to perform a catalytic reaction, a circulation process is allowed.

That is, since the first fluidized bed reactor and the second fluidized bed reactor in which a methane reforming reaction and a reverse Boudouard reaction are performed form circulation process while the yield of the synthetic gas may be maximized through the methane reforming reaction and the reverse Boudouard reaction, a both continuous and economical process operation according to the catalyst regeneration may be implemented.

The reverse Boudouard reaction may be performed by further supplying a carbon source from the outside. Specifically, the carbon source may be char derived from the organic wastes, and more specifically, may be char derived from pyrolysis of waste plastic or biomass-derived char. Otherwise, in order to prevent a possibility of impurity gases flowing in by an external carbon source, high-purity graphite may be included.

In S4), since the catalytic reaction of the present disclosure may proceed to further remove remaining sulfur compounds, nitrogen compounds, and chlorine-based compounds, the yield of the synthetic gas (syngas) may be increased.

The catalyst of S4) includes a first metal including a Group VIIIA element, a Group VIA element, or a combination thereof in the periodic table and a second metal oxide including an alkaline earth metal oxide or a structure thereof. The first metal may include one selected from the group consisting of nickel, iron, molybdenum, and tungsten, and the second metal oxide may include one or more selected from the group consisting of MgO, MgAl₂O₄(Spinel), Mg₆Al₂CO₃(OH)₁₆·4H₂O(Hydrotalcite), and the like.

As described above, since a catalyst including the first metal and second metal oxides is used in the steam reforming process and the reverse Boudouard reaction, the sulfur, nitrogen, and chlorine-based compounds may be selectively further removed, and thus, the production efficiency of the synthetic gas in the steam reforming step and the reverse Boudouard process may be increased. For example, a reaction of removing a sulfur compound by the catalyst including the second metal oxide may be represented by the following Reaction Formula 7:

[Reaction Formula 7] S +CO₂ → SO₂ +SO₃ +CO SO₂ +1/2CO₂ → SO₃ +CO MgO +SO₃ → MgSO₄.

As an example, as shown in Reaction Formula 9, since a catalyst including MgSO₄ produced by a reaction of the second metal oxide with a sulfur compound in S4) is recovered to the first fluidized bed reactor of S2), sulfur is separated from MgSO₄ during the steam reforming reaction to remove impurities and regenerate the catalyst. The reaction may be represented by the following Reaction Formula 8:

[Reaction Formula 8] MgSO₄ + 4H₂ → MgS + 4H₂O MgSO₄ +4H₂ → MgO + H₂S + 3H₂O MgO +H₂O → MgO + H₂S.

As a non-limiting example, a certain amount of catalyst may be replaced with a new catalyst in order to prevent a decrease in catalytic activity by irreversible deactivation of the catalyst which occurs inevitably during the processes of S2) and S4). The partially deactivated catalyst in the steam reforming and reverse Boudouard reaction processes is removed from the reactor, and a new catalyst is introduced to improve catalytic activity. Since the catalyst of the present disclosure has very high durability and a very low deactivation degree of the catalyst, the catalyst replacement cycle is very long, and the amount of a new catalyst which is added for replacement is small.

S5) is a step of producing a synthetic gas from the third mixed gas in which the second stream separated in the carbon dioxide separation unit and carbon monoxide obtained in S4) are mixed through a water gas conversion reaction in a synthetic gas production unit. The third mixed gas may include hydrogen and carbon monoxide.

In the step, the third mixed gas may be converted so that an appropriate hydrogen : carbon monoxide ratio in the catalytic reaction as a subsequent process is satisfied through a water gas conversion (water gas shift, WGS) reaction. The water gas conversion reaction may involve the following Reaction Formula 9:

[Reaction Formula 9] CO + H₂O → H₂ + CO₂.

The water gas conversion reaction may be performed under a catalyst including Fe and Cr. The water gas conversion reaction may be performed at a temperature of 100 to 400°C, specifically 100 to 300°C, and at 20 bar to 80 bar, specifically 25 to 70 bar.

The synthetic gas produced by the water gas conversion reaction may have a ratio of hydrogen : carbon monoxide of 1.5 to 3:1, specifically 1.9 to 2.1:1. Since the ratio of hydrogen : carbon monoxide in the synthetic gas satisfies the range described above, a catalytic reaction process which is a subsequent process may be performed well.

S6) is a step of converting the synthetic gas produced in (S5) into a hydrocarbon oil fraction, in which the synthetic gas may be converted into an appropriate hydrocarbon oil fraction by a catalytic reaction.

The catalytic reaction is not limited as long as it may convert the synthetic gas into a hydrocarbon oil fraction, but specifically, may be a Fischer-Tropsch reaction or a methanol and olefin conversion reaction.

In an example, when the catalytic reaction of S6) is the Fischer-Tropsch reaction, a reaction involving Reaction Formula 10 may be performed using the synthetic gas produced in (S6) as a raw material:

[Reaction Formula 10] nCO + 2nH₂ → CnH₂n + nH₂O.

The Fischer-Tropsch reaction may be performed under a catalyst including cobalt, nickel, or iron, may include alumina, silica, titania, or the like as a support, and may include a precious metal such as Pt, Ru, and Re as a cocatalyst.

The Fischer-Tropsch reaction may be performed at a temperature of 100°C to 500°C, specifically 200°C to 350°C, and at a pressure of 10 atm to 50 atm or 10 atm to 30 atm.

In an example, when the catalytic reaction of (S6) is a methanol and olefin conversion reaction, (S6) may include a reaction of converting the synthetic gas into methanol; and a reaction of converting methanol into olefin.

The reaction of converting the synthetic gas into methanol may involve the following Reaction Formula 11:

[Reaction Formula 11] CO + 2H₂ → CH₃OH.

In an example, the reaction of converting the synthetic gas into methanol may be performed under a Cu-based catalyst. Specifically, the Cu-based catalyst may be a Cu-based methanol-based synthetic catalyst, and as a support of the Cu-based catalyst, one or more selected from the group consisting of SiO₂, ZrO₂, Ga₂O₃ Al₂O₃, MgO, and TiO₂ may be used. Specifically, the Cu-based methanol-based synthetic catalyst may be Cu/Zn/Al₂O₃.

The reaction of converting the synthetic gas into methanol may be performed at 150°C to 600°C, specifically 200°C to 350 °C, and at 0.1 MPa to 10 MPa, specifically 2 MPa to 15 MPa.

The reaction of converting methanol into olefin may be performed under a zeolite-based catalyst or an AlPO₄-based molecular sieve catalyst. Specifically, the zeolite-based catalyst may be ZSM-5, and the AlPO₄-based catalyst may be a silicoaluminaphosphate (SAPO) molecular sieve catalyst, specifically, one or more selected from SAPO-5, SAPO-8, SAPO-11, SAPO-16, SAPO-17, SAPO-18, SAPO-20, SAPO-31, SAPO-34, SAPO-35, SAPO-44, and SAPO-46.

The reaction of converting methanol into olefin may be performed at a temperature of 200°C to 600°C, specifically 300°C to 500°C and at a pressure of 1 bar to 10 bar, specifically 1 bar to 5 bar.

Hereinafter, an apparatus for producing a hydrocarbon of the present disclosure will be described.

The present disclosure includes an apparatus for producing a hydrocarbon including: a pyrolysis reactor which heat treats an organic waste to produce a first mixed gas; a first fluidized bed reactor which steam-reforms the first mixed gas to produce a second mixed gas; a carbon dioxide separation unit which separates the second mixed gas into a first stream including carbon dioxide and a second stream including hydrogen and carbon monoxide; a second fluidized bed reactor which converts the first stream into carbon monoxide through a reverse Boudouard reaction; a gas mixing unit which mixes the second stream and carbon monoxide converted in the second fluidized bed reactor to produce a third mixed gas; a synthetic gas production unit which converts the third mixed gas into a synthetic gas through a water gas conversion reaction; and a hydrocarbon conversion unit which converts the synthetic gas into a hydrocarbon through a catalytic reaction.

Since the apparatus for producing a hydrocarbon 1 according to the present disclosure performs a steam reforming reaction and a reverse Boudouard reaction through a first fluidized bed reactor 20 and a second fluidized bed reactor 40 including a catalyst including a first metal and a second metal oxide, a first mixed gas 110 may be converted into a synthetic gas 230 and a hydrocarbon with high efficiency, and the apparatus may be installed and operated economically with low installation and operating costs.

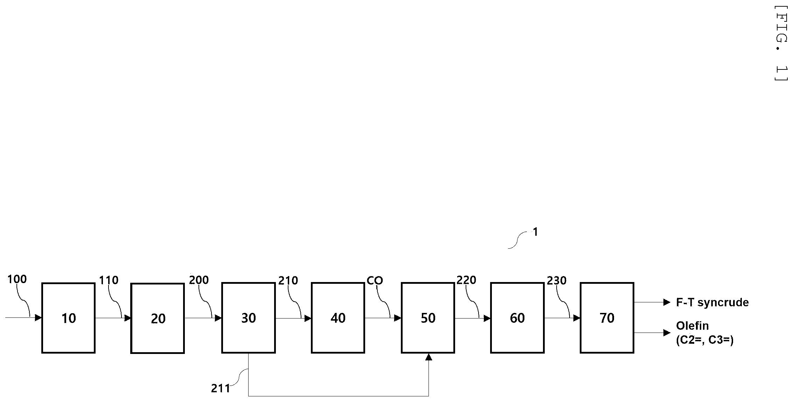

As shown in FIG. 1, the organic waste 100 is introduced into a pyrolysis reactor 10, heat treated and then produces the first mixed gas 110. The first mixed gas 110 is introduced the first fluidized bed reactor 20, and methane is converted into carbon monoxide and hydrogen through a steam reforming reaction to produce a second mixed gas 200.

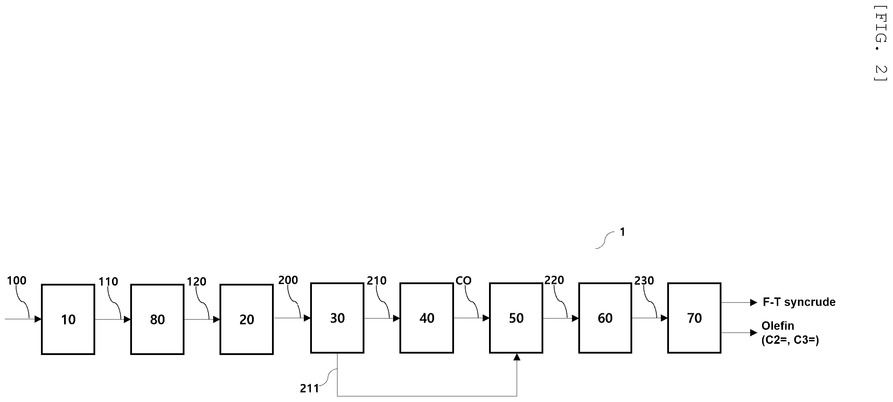

As shown in FIG. 2, a purification unit 80 which removes impurities included in the mixed gas before introducing the mixed gas into the first fluidized bed reactor 20 may be further included. The purification unit 80 may include an adsorbent or a scrubber. A small amount of impurity gas such as S, N, and Cl in the mixed gas is selectively removed through the purification unit 80, thereby decreasing deactivation of the catalyst during steam reforming and reverse Boudouard process operation to increase reaction stability and improve economic feasibility.

The second mixed gas 200 is introduced to a carbon dioxide separation unit 30 and is separated into a first stream 210 including carbon dioxide and a second stream 211 including hydrogen and carbon monoxide. The second stream 211 is supplied directly to a gas mixing unit 50, and the first stream 210 is supplied to the second fluidized bed reactor 40 and converted into carbon monoxide through the reverse Boudouard reaction.

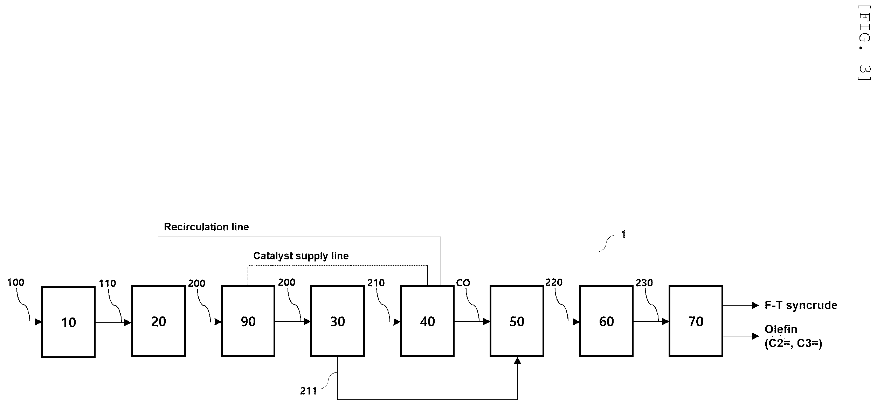

Referring to FIG. 3, a cyclone 90 connected between the first fluidized bed reactor 20 and the carbon dioxide separation unit 30; a catalyst supply line connecting the cyclone 90 and the second fluidized bed reactor 40; and a catalyst recirculation line connecting the second fluidized bed reactor 40 and the first fluidized bed reactor 20 are further included, in which the cyclone 90 may separate the second mixed gas 200 discharged from the first fluidized bed reactor 20 and the catalyst, supply the second mixed gas 200 to the carbon dioxide separation unit 30, and supply the catalyst to the second fluidized bed reactor 4.

In an example, the cyclone 90 may be supplied with the second mixed gas 200 from the first fluidized bed reactor 20, separate a coking catalyst, and then supply the second mixed gas 200 to the separation unit 30, and the coking catalyst may be supplied to the second fluidized bed reactor 40 along a catalyst supply line connected to the cyclone 90 and the second fluidized bed reactor 40. In the second fluidized bed reactor 40, the coking catalyst supplied from the cyclone 90 may be regenerated, and the regenerated catalyst may be supplied to the first fluidized bed reactor 20 again through a recirculation line connected to the fluidized bed reforming reactor 21. As such, when the cyclone 90 is further included, there is no need to separately supply coke required for the reverse Boudouard reaction from the outside, and thus, economic process operation is allowed, and since the catalyst of the methane reforming reaction may be continuously resupplied, efficient process operation is allowed.

The second fluidized bed reactor 40 may be further supplied with char, specifically char derived from an organic waste, and the supplied char and carbon dioxide may react to convert carbon dioxide into carbon monoxide. Without limitation, the char may be char derived from waste plastic pyrolysis or biomass-derived char.

The gas mixing unit 50 mixes the second stream 211 and carbon monoxide converted in the second fluidized bed reactor 40 to produce a third mixed gas 220. The third mixed gas 220 is supplied to the water gas conversion unit 60, and adjusts a ratio of hydrogen : carbon monoxide in the third mixed gas through a water gas conversion reaction to convert it into a synthetic gas 230.

The synthetic gas 230 may be introduced to the hydrocarbon conversion unit 70, converted into a hydrocarbon through a Fisher-Tropsch reaction or a methanol and olefin conversion reaction, and recovered as a high value-added oil fraction.

In an example, when the hydrocarbon conversion unit 70 performs a methanol and olefin conversion reaction, the hydrocarbon conversion unit 70 may include a methanol conversion unit which is supplied with the synthetic gas 230 and converts the synthetic gas into methanol and an olefin conversion unit which is supplied with methanol from the methanol conversion unit and converts methanol into an olefin.

In an example, the apparatus for producing a hydrocarbon may be connected to a hydrocarbon conversion unit 70 and further include a separation step of separating the produced hydrocarbon into fractions at each boiling point through distillation.

Hereinafter, the examples of the present disclosure will be further described with reference to the specific experimental examples. It is apparent to those skilled in the art that the examples and the comparative examples included in the experimental examples are only for illustrating the present disclosure and do not limit the appended claims, and various modifications and alterations of the examples may be made within the range of the scope and spirit of the present disclosure, and these modifications and alterations will fall within the appended claims.

### (Preparation Example 1) Preparation of fluidized bed catalyst (Ni/Mg/Zeolite/Al₂O₃)

10 parts by weight of pseudo-boehmite alumina and 95 parts by weight of nickel nitrate hexahydrate based on 100 parts by weight of water were mixed to prepare a mixed solution. 1 part by weight of formic acid was added while stirring the mixed solution, and the reaction was performed for 3 hours to cause gelation, thereby preparing a precursor gel. Thereafter, 20 parts by weight of clay, 10 parts by weight of USY Zeolite, and 1.5 parts by weight of a MgO were added and mixed with a homogenizer to produce solid mixture, which was mixed with the precursor gel, and then 10 parts by weight of colloidal silica (Ludox AS40, Aldrich) was added, 5 parts by weight of water was further added, and vigorous stirring was performed to prepare a composite catalyst sol. The composite catalyst sol was sprayed and dried to prepare a fluidized bed catalyst having a particle diameter of 50 µm to 250 µm. The recovered catalyst was dried in an oven at 120°C, and fired at 550°C for 3 hours to prepare a fluidized bed catalyst.

### (Preparation Example 2) Preparation of Ni/Mg 3wt%/Zeolite/Al₂O₃ catalyst

A catalyst was prepared in the same manner as in Preparation Example 1, except that the solid mixture included 3 parts by weight of MgO.

### (Preparation Example 3) Preparation of Ni/hydrotalcite/Zeolite/Al₂O₃ catalyst

A catalyst was prepared in the same manner as in Preparation Example 1, except that the solid mixture included hydrotalcite (Mg₆Al₂CO₃(OH)₁₆·4H₂O) instead of MgO.

### (Preparation Example 4) Preparation of Ni/MgSiO₃/Zeolite/Al₂O₃ catalyst

A catalyst was prepared in the same manner as in Preparation Example 1, except that the solid mixture included MgSiO₃ having a spinel structure instead of MgO.

### (Preparation Example 5) Preparation of Ni/Ca/Zeolite/Al₂O₃ catalyst

A catalyst was prepared in the same manner as in Preparation Example 1, except that the solid mixture included CaO instead of MgO.

### (Preparation Example 6) Preparation of Ni/K/Zeolite/Al₂O₃ catalyst

A catalyst was prepared in the same manner as in Preparation Example 1, except that the solid mixture included K₂O instead of MgO.

### (Preparation Example 7) Preparation of Ni/Al₂O₃ catalyst

A catalyst was prepared in the same manner as in Preparation Example 1, except that the solid mixture included 30 parts by weight of clay without including MgO and USY Zeolite.

### (Preparation Example 8) Preparation of Ni/Zeolite/Al₂O₃ catalyst

A catalyst was prepared in the same manner as in Preparation Example 1, except that the solid mixture included 1 part by weight USY Zeolite without including MgO.

### (Experimental Example 1) Analysis of catalyst characteristics

The characteristics of the catalysts prepared by the methods of Preparation Examples 1, 7, and 8 were analyzed and are shown in the following Table 1. Specifically, wear indicators were measured according to the standards of ASTM D 5757-95 using a 3-hole attrition tester. A nickel content in the catalyst was calculated as the results of X-ray fluorescence spectrometry (XRF) analysis, and the XRF analysis was performed using ARL QUANT'X (manufactured by Thermo). A BET surface area, a total pore volume, and a pore size were measured using a Tristar 3000 instrument (manufactured by Micromeritics) by a nitrogen physical adsorption-desorption method. A particle size and a particle size distribution such as a fine particle content included in the catalyst were measured using Mastersizer 3000 (manufactured by Malvern). Herein, "fine particle" refers to a catalyst particle having a particle diameter of 50 µm or less.

**[Table 1]**

| | Preparation Example 1 | Preparation Example 7 | Preparation Example 8 |
|---|---|---|---|
| Wearing indicator (wt%) | 3.1 | 3.5 | 5.3 |
| BET surface area (m2/g) | 124 | 79 | 129 |
| Total pore volume (cc/g) | 0.23 | 0.2 | 0.23 |
| Pore average particle diameter (Å) | 48 | 34 | 49 |
| Average particle diameter (µm) | 127 | 126 | 121 |
| Fine particle content in catalyst (wt%) | 3.5 | 4 | 5 |
| Ni content in catalyst (wt%) | 29.1 | 30.1 | 28.5 |

As shown in Table 1, the catalyst of Preparation Example 1 including nickel and MgO had a low wearing indicator of 3.1 wt%, but the catalysts of Preparation Examples 7 and 8 including no MgO had the wearing indicator of 3.5 wt% and 5.3 wt%, respectively. In addition, the fine particle content included in the catalysts of Preparation Examples 1, 7, and 8 was measured as 3.5 wt%, 4 wt%, and 5 wt%, respectively. It was found that the catalyst of Preparation Example 1 of which the measured fine particle content and wearing indicator were the lowest had excellent strength. Besides, since it was measured that the catalyst of Preparation Example 1 had a specific surface area of 124 m²/g, a pore volume of 0.23 cc/g, a pore average particle diameter of 48 Å, and a catalyst average particle diameter of 127 µm, the catalyst was confirmed to have a structure which may improve catalytic activity by sufficiently dispersing and supporting nickel and MgO on a ceramic support.

### (Example 1)

1000 g of a municipal solid waste was added to a pyrolysis reactor, water vapor was introduced at a temperature of 1200°C and a pressure of 250 kPa under an alumina bed, a heat treatment was performed, and a first mixed gas having a C/O element ratio of 0.67 was recovered.

The temperature of the first mixed gas was lowered, impurities such as Cl, S, and N included in the mixed gas were removed through a scrubber, and a purified first mixed gas was recovered.

The first mixed gas with impurities removed was supplied to a first fluidized bed reactor including the catalyst prepared in Preparation Example 1 at a spatial velocity of 2 L/g-cat./h, water vapor was supplied simultaneously, and a second mixed gas was prepared by steam reforming at 700°C.

The second mixed gas was introduced to an amine scrubber, carbon dioxide in the second mixed gas was captured in the amine scrubber, and a second stream from which carbon dioxide had been separated was separated. Specifically, the second mixed gas was introduced to the first amine scrubber to capture CO₂ in an aqueous solution (amine solution) in which monoethanolamine (MEA) was dissolved at 50°C, and uncaptured gas was recovered as a second stream. The amine solution in the first amine scrubber was introduced to the second amine scrubber and separated into an amine solution and CO₂ at 100°C, and CO₂ was recovered as a first stream.

The recovered first stream was introduced to the second fluidized bed reactor including the catalyst of Preparation Example 1 and converted into carbon monoxide through a reverse Boudouard reaction. The reverse Boudouard reaction was performed in a second fluidized bed reactor filled with the catalyst of Preparation Example 2, which had been used in the steam reforming reaction and had coke deposited thereon, and the first stream was continuously supplied at a spatial velocity of 2 L/g-cat./h and converted into carbon monoxide. The catalyst activated by removing coke through the reverse Boudouard reaction was added again to the first fluidized bed reactor.

The catalyst separated from the cyclone was supplied to the second fluidized bed reactor through a catalyst supply line and used as a carbon source, and also treated together when performing the reverse Boudouard reaction to perform a regeneration process. Further, the regenerated catalyst was resupplied to the first fluidized bed reactor through a recirculation line connected to the first fluidized bed reactor, and unconverted CO₂ after the reaction was separately recovered through the amine scrubber.

The carbon monoxide and the second stream converted from the first stream were introduced to a gas mixing unit, and mixed at 200°C to produce a third mixed gas.

The third mixed gas was introduced to a synthetic gas production unit to produce the synthetic gas having a mole ratio of H₂:CO=1:2 through a water gas conversion reaction.

The synthetic gas was supplied to a hydrocarbon conversion unit, an injection speed was set so that a spatial velocity was 5000L/kg· cat/h and at a volume ratio of carbon monoxide: hydrogen: argon was 63.2: 31.3: 5.5 under a Co/ZnO (cobalt zinc oxide) catalyst, and a Fischer-Tropsch reaction was performed at a reaction temperature of 300°C and a pressure of 10 bar for 60 hours to recover a hydrocarbon oil fraction.

### (Example 2)

The process was performed in the same manner as in Example 1, except that the catalyst of Preparation Example 2 was used instead of the catalyst of Preparation Example 1, as the catalyst of the steam reforming process and the reverse Boudouard reaction process.

### (Example 3)

The process was performed in the same manner as in Example 1, except that the catalyst of Preparation Example 3 was used instead of the catalyst of Preparation Example 1, as the catalyst of the steam reforming process and the reverse Boudouard reaction process.

### (Example 4)

The process was performed in the same manner as in Example 1, except that the catalyst of Preparation Example 4 was used instead of the catalyst of Preparation Example 1, as the catalyst of the steam reforming process and the reverse Boudouard reaction process.

### (Example 5)

The process was performed in the same manner as in Example 1, except that the catalyst of Preparation Example 5 was used instead of the catalyst of Preparation Example 1, as the catalyst of the steam reforming process and the reverse Boudouard reaction process.

### (Example 6)

The process was performed in the same manner as in Example 1, except that the catalyst of Preparation Example 6 was used instead of the catalyst of Preparation Example 1, as the catalyst of the steam reforming process and the reverse Boudouard reaction process.

### (Comparative Example 1)

The process was performed in the same manner as in Example 1, except that the catalyst of Preparation Example 7 was used instead of the catalyst of Preparation Example 1 in the steam reforming process and the reverse Boudouard reaction process.

### (Comparative Example 2)

The process was performed in the same manner as in Example 1, except that the catalyst of Preparation Example 8 was used instead of the catalyst of Preparation Example 1 in the steam reforming process and the reverse Boudouard reaction process.

### (Experimental Example 2) Evaluation of catalyst durability

A process of producing a synthetic gas from the first mixed gas in the preparation of a hydrocarbon by the methods of Examples 1 to 6 and Comparative Examples 1 and 2 was defined as one cycle, 20 cycles were performed, the catalyst was taken out of the reactor, the particle size distribution of the catalyst was analyzed, and the fine particle contents in the catalyst were compared. At this time, the fine particles refer to particles having a catalyst particle diameter of 50 µm or less.

The reactor was filled with 2 kg of the catalyst, 3 cc/min of water was introduced at 750°C, a first mixed gas obtained by pyrolyzing wastes was supplied, and a methane steam reforming reaction was performed. The first mixed gas included 99.5% of CH4, and more specifically, the type and content of impurities included in the first mixed gas are shown in the following Table 2.

**[Table 2]**

| Impurity | contents (ppm) |
|---|---|
| HCl | 200 |
| H₂S | 100 |
| COS | 20 |
| NH₃ | 350 |

The particle size distribution of the catalyst remaining in the reactor after performing the reaction for 20 cycles was analyzed, and the fine particle contents in the catalyst were measured and are shown in the following Table 3. The particle size distribution was measured using Mastersizer 3000 (manufactured by Malvern).

**[Table 3]**

| | Type of catalyst | Fine particle content (wt%) |
|---|---|---|
| Example 1 | Preparation Example 1 (Ni/Mg 1 wt%/Zeolite/Al₂O₃) | 12.8 wt% |
| Example 2 | Preparation Example 2 (Ni/Mg 3wt%/Zeolite/Al₂O₃) | 11.2 wt% |
| Example 3 | Preparation Example 3 (Ni/Hydrotalcite/Zeolite/Al₂O₃) | 14.7 wt% |
| Example 4 | Preparation Example 4 (Ni/MgSiO₃/Zeolite/Al₂O₃) | 14.0 wt% |
| Example 5 | Preparation Example 5 (Ni/Ca/Zeolite/Al₂O₃) | 14.1 wt% |
| Example 6 | Preparation Example 6 (Ni/K/Zeolite/Al₂O₃) | 14.9 wt% |
| Comparative Example 1 | Preparation Example 7 (Ni/Al₂O₃) | 17.5 wt% |
| Comparative Example 2 | Preparation Example 8 (Ni/Zeolite/Al₂O₃) | 22.7 wt% |

As shown in Table 3, Examples 1 to 6 using the catalyst of Preparation Examples 1 to 7 including the first metal and the second metal oxide had lower ratios of catalyst converted into fine particles than Comparative Examples 1 and 2 including the catalyst of Preparation Examples 7 and 8 including no second metal oxide. The increased fine particles mean that cracks and breaks occurred in the catalyst by repeating the catalyst coking and catalyst regeneration for 20 cycles. When two types of metals were included as in Preparation Examples 1 to 6, the catalyst maintained its original state without damaging the catalyst even with the repeated performance of the catalyst coking and catalyst regeneration process, and thus, the circulation process was able to proceed continuously for a long time. Therefore, since the catalyst of the present disclosure had excellent durability and an improved catalyst lifespan, a catalyst exchange cycle was extended, and process efficiency was improved.

### (Experimental Example 3) Evaluation of catalyst performance

The catalysts of Preparation Examples 1 to 8 were operated for one cycle, 5 cycles, and 20 cycles by the methods of Examples 1 to 6 and Comparative Examples 1 and 2, respectively, in the same manner as in Experimental Example 2, and 200 g of the catalysts were recovered each time. Fine particles were removed from the recovered catalyst, and a reforming reaction of a methane gas including impurities was performed in a separate reactor.

A methane reforming reaction was performed by filling a fluidized bed reactor with 100 g of the catalyst, performing catalyst reduction at a temperature of 900°C for 2 hours under the conditions of a H₂ gas flow rate of 3.03 Nl/min, and then performing a methane reforming reaction under the conditions of a total gas flow rate of 3.33 Nl/min, a processing amount of 2.0 L/gcat·h, and CH₄/H₂O = 3. The methane conversion rates of the catalysts are shown in the following Table 4. In Table 4, "Fresh cat." refers to a new catalyst without performing a catalytic reaction.

**[Table 4]**

| | | Type of catalyst | | Methane conversion rate (%) | |
|---|---|---|---|---|---|
| | | Fresh cat. | 1 cycle | 5 cycles | 20 cycles |
| Example 1 | Preparation Example 1 (Ni/Mg 1wt%/Zeolite/Al₂O₃) | 98.3 | 85.2 | 65.3 | 23.5 |
| Example 2 | Preparation Example 2 (Ni/Mg 3wt%/Zeolite/Al₂O₃) | 97.0 | 83.2 | 53.1 | 17.2 |
| Example 3 | Preparation Example 3 (Ni/Hydrotalcite/Zeolite/Al₂O₃) | 97.5 | 79.9 | 35.9 | 9.5 |
| Example 4 | Preparation Example 4 (Ni/MgSiO₃/Zeolite/Al₂O₃) | 97.3 | 77.1 | 29.2 | 8.8 |
| Example 5 | Preparation Example 5 (Ni/Ca/Zeolite/Al₂O₃) | 97.5 | 75.0 | 27.1 | 5.9 |
| Example 6 | Preparation Example 6 (Ni/K/Zeolite/Al₂O₃) | 93.3 | 79.3 | 14.0 | 3.1 |
| Comparative Example 1 | Preparation Example 7 (Ni/Al₂O₃) | 99.3 | 73.9 | 22.3 | 3.0 |
| Comparative Example 2 | Preparation Example 8 (Ni/Zeolite/Al₂O₃) | 99.0 | 78.3 | 15.5 | 4.1 |

Referring to Table 4, Comparative Examples 1 and 2 using the catalysts of Preparation Examples 7 and 8 which did not include the alkaline earth metal or a structure thereof had very high initial catalytic activity, but a methane conversion rate after 5 cycles of 22.3% and 15.5%, respectively, which were significantly decreased as compared with the initial methane conversion rate, and it was confirmed that the catalyst was rapidly deactivated as the catalyst process was repeatedly performed. Even the methane conversion rates after 20 cycles were decreased to 3.0% and 4.1% in Comparative Examples 1 and 2, respectively, and it was confirmed that most of the catalyst particles were deactivated.

However, Examples 1 to 6 using the catalysts of Preparation Examples 1 to 6 containing nickel and an alkaline earth metal showed high catalytic activity even with the repeated performance of catalyst coking and regeneration. In particular, Examples 1 and 2 including MgO had high methane conversion rates of 65.3% and 53.1%, respectively, even after 5 cycles and 23.5% and 17.2%, respectively, even after 20 cycles, and their deactivation of the catalyst was delayed. Even in Examples 3 and 4 using the catalysts of Preparation Examples 3 and 4 including hydrotalcite which is an alkaline earth metal structure or MgSiO₃ having a spinel structure, the methane conversion rate was measured as 35.9% and 29.2% after 5 cycles, respectively, and measured as good as 9.5% and 8.8%, respectively, even after 20 cycles. The catalyst of Preparation Example 5 using CaO as the alkaline earth metal oxide had the methane conversion rate after 5 cycles and 20 cycles measured as 27.1% and 5.9%, respectively.

Meanwhile, Example 6 including the catalyst of Preparation Example 6 including K₂O which is an alkali metal oxide had a decreased methane conversion rate after 5 cycles of 14.0% and a decreased methane conversion rate after 20 cycles of 3.1%, and it was confirmed that catalyst deactivation was not suppressed.

It was confirmed from the examples and the comparative examples that when a hydrocarbon was produced from the pyrolysis gas by the method according to the present disclosure, since carbon dioxide in the mixed gas was recovered and converted into carbon monoxide by a reverse Boudouard reaction, the yields of synthetic gas and hydrocarbon were increased, the amounts of H₂ and CO produced of the final synthetic gas were significantly increased, a CO₂ production amount was reduced, and thus, the catalyst had a beneficial effect in terms of synthetic gas production yield and prevention of environmental pollution. In particular, since a fluidized bed reactor was adopted as the reactor of the methane reforming and reverse Boudouard reaction process, and the catalyst having excellent durability by complexing the second metal oxide including the first metal and the alkaline earth metal was used as the catalyst of the methane reforming and the reverse Boudouard reaction, catalyst deactivation due to coke produced during the methane reforming reaction was prevented, and even when the catalyst was deactivated with coke, the catalyst was able to be regenerated in the reverse Boudouard reaction process. Thus, the catalyst exchange cycle was extended to improve process efficiency, and furthermore, excellent synthetic gas conversion rate and hydrocarbon production efficiency were able to be achieved. In addition, since the steam reforming reaction and the reverse Boudouard reaction were performed while continuously removing impurities in the mixed gas through the catalyst, high-purity synthetic gas was able to be obtained.

The above description is only an example to which the principle of the present disclosure is applied, and other constitutions may be further included without departing from the scope of the present disclosure.

**[Detailed Description of Main Elements]**

| | | | |
|---|---|---|---|
| 1 | Apparatus for producing a hydrocarbon | | |
| 10 | Pyrolysis reactor | | |
| 20 | First fluidized bed reactor | 30 | Carbon dioxide separation unit |
| 40 | Second fluidized bed reactor | 50 | Gas mixing unit |
| 60 | Synthetic gas production unit | 70 | Hydrocarbon conversion unit |
| 80 | Purification unit | 90 | Cyclone |
| 100 | Organic waste | 110 | First mixed gas |
| 120 | First mixed gas with impurities removed | 200 | Second mixed gas |
| 210 | First stream | 211 | Second stream |
| 220 | Third mixed gas | 230 | Synthetic gas |

## Claims

1. A method for producing a hydrocarbon, the method comprising:
S1) pyrolyzing a mixed waste to produce a first mixed gas;
S2) steam-reforming the first mixed gas from which impurities have been removed in a first fluidized bed reactor to produce a second mixed gas;
S3) separating the second mixed gas into a first stream including carbon dioxide and a second stream including hydrogen and carbon monoxide;
S4) introducing the first stream separated in S3) into a second fluidized bed reactor and converting the first stream into carbon monoxide through a reverse Boudouard reaction;
S5) mixing the second stream and the carbon monoxide converted in S4) to produce a third mixed gas and treating the third mixed gas through a water gas conversion reaction to produce a synthetic gas (syngas); and
S6) producing a hydrocarbon from the synthetic gas through a catalytic reaction.

2. The method for producing a hydrocarbon of claim 1,
wherein S2) and S4) are performed under a catalyst containing a first metal including a Group VIIIA element, a Group VIA element, or a combination thereof; and
a second metal including an alkaline earth metal oxide or an alkaline earth metal oxide structure.

3. The method for producing a hydrocarbon of claim 1, wherein the first mixed gas further includes one or more selected from the group consisting of landfill gas, shale gas, refinery exhaust gas, and biogas.

4. The method for producing a hydrocarbon of claim 1, wherein the second fluidized bed reactor in S4) is supplied with a coking catalyst of the first fluidized bed reactor of S2) as a carbon source.

5. The method for producing a hydrocarbon of claim 1, wherein the first stream includes 50 vol% or more of carbon dioxide.

6. The method for producing a hydrocarbon of claim 1, wherein the pyrolysis gas has a C/O element ratio of 0.1 or more.

7. The method for producing a hydrocarbon of claim 1, wherein S2) is performed at a temperature of 700°C to 1000°C.

8. The method for producing a hydrocarbon of claim 1, wherein S4) is performed at temperature of 600°C to 1000°C and a pressure of 50 kPa to 300 kPa.

9. The method for producing a hydrocarbon of claim 1, wherein in S5), the synthetic gas includes hydrogen and carbon monoxide and a ratio between the hydrogen and the carbon monoxide satisfies 1.8:1 to 2.2:1.

10. The method for producing a hydrocarbon of claim 1, wherein in S1), the organic waste is one or more selected from the group consisting of waste plastic, solid waste, biomass, waste oil, waste tire, and standard plastic garbage bags.

11. An apparatus for producing a hydrocarbon comprising:
a pyrolysis reactor which heat treats an organic waste to produce a first mixed gas;
a first fluidized bed reactor which steam-reforms the first mixed gas to produce a second mixed gas;
a carbon dioxide separation unit which separates the second mixed gas into a first stream including carbon dioxide and a second stream including hydrogen and carbon monoxide;
a second fluidized bed reactor which converts the first stream into carbon monoxide through a reverse Boudouard reaction;
a gas mixing unit which mixes the second stream and carbon monoxide converted in the second fluidized bed reactor to produce a third mixed gas;
a synthetic gas production unit which converts the third mixed gas into a synthetic gas through a water gas conversion reaction; and
a hydrocarbon conversion unit which converts the synthetic gas into a hydrocarbon through a catalytic reaction.

12. The apparatus for producing a hydrocarbon of claim 11, wherein the first fluidized bed reactor and the second fluidized bed reactor include a catalyst containing a first metal including a Group VIIIA element, a Group VIA element, or a combination thereof in the periodic table; and a second metal including an alkaline earth metal oxide or an alkaline earth metal oxide structure.

13. The apparatus for producing a hydrocarbon of claim 11, further comprising:
a cyclone connected between the first fluidized bed reactor and the carbon dioxide separation unit;
a catalyst supply line which connects the cyclone and the second fluidized bed reactor; and
a catalyst recirculation line which connects the second fluidized bed reactor and the first fluidized bed reactor,
wherein the cyclone separates the second mixed gas and the catalyst discharged from the fluidized bed reforming reactor, supplies the second mixed gas to the carbon dioxide separation unit, and supplies the catalyst to the second fluidized bed reactor.
